# EUROPÄISCHE PATENTANMELDUNG

(11) **EP 0 940 398 A1**
(43) Veröffentlichungstag der Anmeldung: **08.09.1999**
(21) Anmeldenummer: 99101902.7
(22) Anmeldetag: 29.01.1999
(51) Int. Cl.: C07D 321/00

(54) **Verfahren zur Herstellung von makrocyclischen Estern**

(30) Priorität: 03.03.1998 DE 19808843
(71) Anmelder: HÜLS AKTIENGESELLSCHAFT, 45764 Marl (DE)
(72) Erfinder: Köhler, Günter Dr., 45770 Marl (DE); Feld, Marcel Dr., 51147 Köln (DE); Osterholt, Clemens, 46286 Dorsten (DE); Metz, Josef Dr., 45770 Marl (DE)
(74) Vertreter: Olbricht, Gerhard, Dr.

(57) **Zusammenfassung**

Die Erfindung betrifft ein Verfahren zur Herstellung von makrocyclischen Estern der allgemeinen Formel 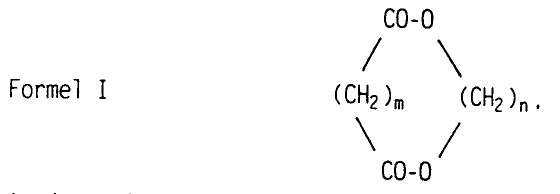 in der m die Bedeutung einer ganzen Zahl von 6 bis 14 hat und n für eine ganze Zahl von 2 bis 6 steht, dadurch gekennzeichnet, daß man
(a) Dicarbonsäure-bis(glykol)ester der allgemeinen Formeln

   HO-(CH₂)ₙ-O-CO-(CH₂)ₘ-CO-O-(CH₂)ₙOH, Formel II

   H[O-(CH₂)ₙ-O-CO-(CH₂)ₘ-CO-]ₓ-O-(CH₂)ₙ-OH, Formel III

   H[O-(CH₂)ₙ-O-CO-(CH₂)ₘ-CO-]ₓ-OH Formel IV

   und/oder

   HO-CO-(CH₂)ₘ-CO-[-O-(CH₂)ₙO-CO-(CH₂)ₘ-CO-]ₓ-OH, Formel V

   in denen m und n die für die Formel I angegebenen Bedeutungen haben und x für eine ganze Zahl >1 steht,
(b) ein Glykol der allgemeinen Formel Formel VI, HO-(CH₂)ₙ-OH, in der 20 n denselben numerischen Wert wie in den Formeln I bis V hat, in der 1- bis 50-fachen molaren Menge, bezogen auf den Ester II und die Dicarbonsäurebausteine der Ester III bis V, und
(c) ein inertes hochsiedendes Reaktionsmedium in der 0,1 bis 20-fachen Gewichtsmenge, bezogen auf die Summe der Gewichtsmengen der Ester II bis V, in Gegenwart (d) eines Katalysators
in einem Verdampfer mit großer Oberfläche auf Temperaturen von 150 bis 350°C bei einem vermindertem Druck von etwa 0,1 bis etwa 500 mbar erhitzt, wodurch unter Abspaltung von Glykol der makrocyclische Ester I entsteht, der zusammen mit Glykol VI abdestilliert und durch Kondensation gewonnen wird.

## Beschreibung

Die Erfindung betrifft ein Verfahren zur Herstellung makrocyclischer, vorzugsweise 12- bis 20-gliedriger Ester der allgemeinen Formel 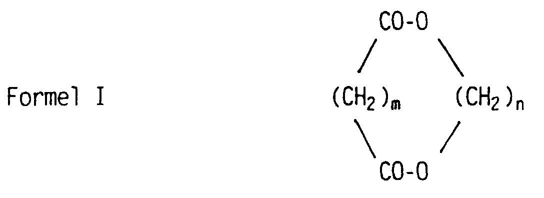 in der m die Bedeutung einer ganzen Zahl von 6 bis 14 hat und n für eine ganze Zahl von 2 bis 12 steht, aus Dicarbonsäure-glykolestern der allgemeinen Formeln

HO-(CH₂)ₙ-O-CO-(CH₂)ₘ-CO-O-(CH₂)ₙ-OH Formel II

H[O-(CH₂)ₙ-O-CO-(CH₂)ₘ-CO-]ₓ-O-(CH₂)ₙ-OH Formel III

H[O-(CH₂)ₙ-O-CO-(CH₂)ₘ-CO-]ₓ-OH Formel IV

und/oder

HO-CO-(CH₂)ₘ-CO-[-O-(CH₂)ₙO-CO-(CH₂)ₘ-CO-]ₓ-OH, Formel V

wobei m und n die oben angegebene Bedeutung haben und x für eine ganze Zahl >1, vorzugsweise von 2 bis 10 steht.

Cyclische Ester dieser Art und insbesondere das formal aus den monomeren Bausteinen Brassylsäure und Etylenglykol gebildete cyclische Ethylenbrassylat haben in der Parfümindustrie als Duftbestandteil mit Ambra- oder Moschusnote bzw. als Fixateur in Riechstoffmischungen eine herausragende Bedeutung.

Es ist bekannt, makrocyclische Ester durch cyclisierende Depolymerisation oligomerer oder polymerer Glykolester entsprechender Dicarbonsäuren zu gewinnen. Üblicherweise wird die Depolymerisation bei hohen Temperaturen und unter vermindertem Druck so durchgeführt, daß die dabei gebildeten Zielprodukte abdestillieren und durch Kondensation gewonnen werden können. Die cyclisierende Depolymerisation ist z.B. in J.Am.Chem.Soc. **57** (1935), 929-34 und in US-A-4,175,321 beschrieben.

Es ist weiter aus US-A 4 709 058, JA-AS 55-120 581 und DE 32 25 341 bekannt, daß bei der Herstellung von makrocyclischen Estern durch cyclisierende Depolymerisation die Mitverwendung von inerten hochsiedenden Reaktionsmedien von Vorteil ist. Ein wesentliches Problem bei jeder cyclisierenden Depolymerisation besteht darin, daß unter den Reaktionsbedingungen auch höhermolekulare Ester gebildet werden können, indem Oligomere oder Polymere mit endständigen Carboxylgruppen mit anderen Oligomeren oder Polymeren, die endständige Hydroxylgruppen tragen, unter Abspaltung von Wasser polykondensieren oder indem Oligomere oder Polymere mit endständigen Hydroxyalkylgruppen unter Glykolabspaltung polykondensieren. Die erwünschte intramolekulare Bildung der makrocyclischen monomeren Zielprodukte wird also von einer unerwünschten intermolekularen Bildung linearer, reaktionsträger hochpolymerer Ester begleitet. Dies aber vermindert nicht nur die Ausbeute an Zielprodukt, sondern wirft auch erhebliche verfahrenstechnische Probleme auf.

Bei den bisher beschriebenen Verfahren wird die Reaktion im allgemeinen batchweise oder semikontinuierlich durchgeführt. Dies ist bei einer Synthese in kleiner Größenordnung, etwa im Labor- oder Technikumsmaßstab, völlig unproblematisch. Die Übertragung des Verfahrens in den technischen Maßstab wirft jedoch Probleme auf, wobei insbesondere eine batchweise oder kontinuierliche Reaktionsführung im Rührkessel beträchtliche Nachteile aufweist. Durch die gebildeten höhermolekularen Produkte sinkt nämlich die Wärmeleitfähigkeit des Reaktionsgemisches, während gleichzeitig die Viskosität ansteigt. Dadurch wird das Abdestillieren der monomeren Zielprodukte erschwert, was wiederum die Bildung höhermolekularer Produkte begünstigt. Deren Anteil steigt also an, wodurch in zunehmendem Maße Sumpfprodukt entsteht, das entsorgt werden muß. Wird die Reaktion nicht rechtzeitig abgebrochen, ist sogar ein Erstarren des gesamten Kesselinhalts möglich.

Darüber hinaus ist es technisch nicht einfach, die für die cyclisierende Depolymerisation erforderliche Energiemenge in einen Rührkessel einzubringen und zugleich dem Zielprodukt sowie dem zwangsläufig mit dem Zielprodukt abdestillierenden gebildeten und gegebenenfalls zusätzlich eingebrachten Glykol eine möglichst große Verdampfungsfläche zu bieten.

Dem wird nach der europäischen Patentanmeldung 929 07 653.7 durch die Verwendung eines speziellen, horizontalen Dünnschichtverdampfers Rechnung getragen, der jedoch bei sehr hohen Temperaturen von >300°C und mit praktisch unverdünntem polymerem Einsatzprodukt betrieben werden muß und bei dem die Probleme durch Bildung hochpolymerer Produkte besonders gravierend sein können.

Die genannten Probleme werden durch das erfindungsgemäße Verfahren auf einfache, vorteilhafte und technisch leicht realisierbare Weise gelöst. Gegenstand der Erfindung ist ein Verfahren zur Herstellung von makrocyclischen, vorzugsweise 12 bis 20 Ringglieder umfassenden Estern der allgemeinen Formel 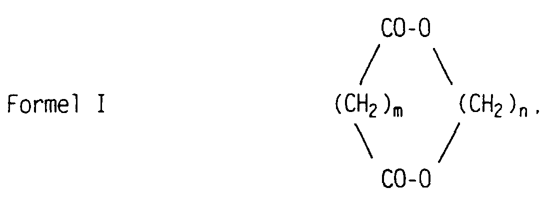 in der m die Bedeutung einer ganzen Zahl von 6 bis 14 hat und n für eine ganze Zahl von 2 bis 12 steht, wobei man
(a) Dicarbonsäure-glykolester der allgemeinen Formeln

   HO-(CH₂)ₙ-O-CO-(CH₂)ₘ-CO-O-(CH₂)ₙ-OH, Formel II

   H[O-(CH₂)ₙ-O-CO-(CH₂)ₘ-CO-]ₓ-O-(CH₂)ₙ-OH, Formel III

   H[O-(CH₂)ₙ-O-CO-(CH₂)ₘ-CO-]ₓ-OH Formel IV

   und/oder

   HO-CO-(CH₂)ₘ-CO-[-O-(CH₂)ₙO-CO-(CH₂)ₘ-CO-]ₓ-OH, Formel V

   in denen m und n die für die Formel I angegebenen Bedeutungen haben und x für eine ganze Zahl >1, vorzusweise von 2 bis 10 steht.
(b) ein Glykol der allgemeinen Formel

   HO-(CH₂)ₙ-OH, Formel VI

   in der n denselben numerischen Wert wie in den Formeln I bis V hat, in der 1- bis 50-fachen, vorzugsweise in der 2- bis 20-fachen molaren Menge Menge, bezogen auf den Ester V und die Dicarbonsäurebausteine der Ester II bis IV, und
(c) ein inertes hochsiedendes Reaktionsmedium in der 0,1 bis 20-fachen, vorzugsweise in der 1- bis 15-fachen und insbesondere in der 2- bis 10-fachen Gewichtsmenge, bezogen auf die Summe der Gewichtsmengen der Ester II bis V, in Gegenwart
(d) eines Katalysators
in einem Verdampfer mit großer Oberfläche auf Temperaturen von 150 bis 350°C, vorzugsweise von 180 bis 300°C und insbesondere von 200 bis 280°C. bei einem vermindertem Druck von etwa 0,1 bis etwa 500 hPa, vorzugsweise von 0,5 bis 100 hPa erhitzt, wodurch unter Abspaltung von Glykol der makrocyclische Ester I entsteht, der zusammen mit Glykol VI abdestilliert und durch Kondensation gewonnen wird.

Überraschendenweise erhält man bei dem erfindungsgemäßen Verfahren Ausbeuten an makrocyclischem Ester I von >90 % d.Th. trotz der Anwesenheit von überschüssigem Glykol, das das Umesterungsgleichgewicht in Richtung auf den monomeren Dicarbonsäure-bis(glykol)ester V verschiebt, also die Cyclisierungsreaktion zurückdrängt.

Das erfindungsgemäße Verfahren wird vorteilhaft kontinuierlich durchgeführt, wobei das inerte hochsiedende Medium, das den Katalysator und nicht umgesetzte Ester II bis V enthält, im Kreis geführt wird. Bevorzugt wird das kontinuierliche Verfahren so durchgeführt, daß man die Lösung der Dicarbonsäure-glykolester II bis V in dem entsprechenden Glykol, die den Katalysator enthält, in das rückgeführte inerte hochsiedende Medium einbringt und die entstehende Mischung in die Verdampfungszone einführt, die zugleich Reaktionszone ist und aus der der gebildete makrocyclische Ester I und Glykol VI abdestillieren. Die Edukte, d.h. die Ester II bis V, werden in nur einem Durchgang weitgehend umgesetzt. Bei optimaler Fahrweise verbleiben daher nur 1 bis 10 Gew.-% Edukt, bezogen auf das inerte hochsiedende Medium, unverdampft zurück und werden nach Anreicherung mit frischem Edukt und Glykol in die Verdampfungszone zurückgeführt. Die Verweilzeit in dem Verdampfer mit großer Oberfläche beträgt pro Durchgang vorteilhaft 0,5 bis 10 Minuten. Sie wird durch die Leistung der Pumpe geregelt, die den Kreislauf bewirkt. Nach Erreichen des stationären Zustandes werden bis zu 100% der Edukte in das Zielprodukt I überführt. Im Verlauf einer längeren Produktionskampagne, die eine Anlaufphase und den stationären Zustand umfaßt, werden somit - je nach Länge der Kampagne - weit über 95% der Edukte in das Zielprodukt I umgewandelt.

Die Ester II bis V leiten sich von Dicarbonsäuren und Glykolen (oder Diolen) ab. Geeignete Dicarbonsäuren haben beispielsweise 2 bis 20, vorzugsweise 4 bis 12 Kohlenstoffatome zwischen den Carboxylgruppen. Beispiele hierfür sind u.a. Bernsteinsäure, Adipinsäure, Korksäure, Sebacinsäure (1,10-Decansäure), 1,12-Dodecandisäure und Brassylsäure (1,13-Tridecandisäure). Von den geeigneten Glykolen VI, die z.B. 2 bis 12 Kohlenstoffatome zwischen den Hydroxylgruppen enthalten können, seien z.B. Ethylenglykol, Ethylendiglykol, 1,3-Propandiol, 1,4-Butandiol, 1,6-Hexandiol, 1,8-Octandiol und 1,12-Dodecandiol genannt.

Eine für die Durchführung des erfindungsgemäßen Verfahrens geeignete glykolische Lösung der Ester II bis V kann beispielsweise unter Verwendung üblicher Veresterungs- bzw. Umesterungskatalysatoren durch direkte Veresterung der Dicarbonsäure mit dem Glykol VI, durch Umesterung eines Dicarbonsäuredialkylesters eines aliphatischen, niedermolekularen Alkohols mit vorzugsweise 1 bis 6 Kohlenstoffatomen mit dem Glykol VI unter Abspaltung des aliphatischen niedermolekularen Alkohols oder durch Depolymerisation eines hochpolymeren Esters der Formeln III bis V, in denen x z.B. >10 ist, mit überschüssigem Glykol VI hergestellt werden. Im letzteren Fall werden also hochpolymere Ester III bis V zu einem Gemisch aus monomerem Ester II und höhermolekularem Ester III mit einem kleineren Wert von x, vorteilhaft von 2 bis 10, abgebaut (die Entstehung von höhermolekularen Estern IV und V, die freie Carboxylgruppen enthalten, ist wegen des Glykolüberschusses nicht begünstigt). In allen drei genannten Fällen wird die Bildung des monomeren Dicarbonsäure-bis(glykol)esters II mit zunehmendem Überschuß an Glykol VI begünstigt. Wenn man die Menge des Glykols VI begrenzt, z.B. auf höchstens 20 Mol Glykol je Mol Dicarbonsäure bzw. Dicarbonsäurebaustein, erhält man Edukte II bis V, die verhältnismäßig arm an monomerem Ester II und reich an höherem Dicarbonsäureester III sind. Lösungen, die praktisch keine monomeren Ester II enthalten, lassen sich nur mit sehr geringen Überschüssen an Glykol VI herstellen. Praktisch wird man stets Ester II bis V nebeneinander vorliegen haben. Beispielsweise kann man mit gutem Erfolg Lösungen der Ester II bis V einsetzen, in denen die Ester III bis V in Mengen von 70 bis 95 Gew.-% vorliegen, bezogen auf die Summe der Ester II bis V. Da man bei allen beschriebenen Herstellungsweisen eine Mischung von Molekülen mit unterschiedlichem Oligomerisierungsgrad erhält, ist x für die Lösung immer ein Mittelwert.

Wenn man die glykolischen Lösungen der Ester II bis V auf die beschriebene Weise herstellt, enthalten sie in der Regel die erforderlichen Mengen Glykol VI. Wenn dies nicht der Fall ist oder eine an sich ausreichende Menge zwecks Optimierung noch vergrößert werden soll, wird weiteres Glykol zugesetzt. Eine Optimierung kann beispielsweise erfolgen, wenn bei der Herstellung der glykolischen Lösung der Ester II bis V im Interesse einer hohen Raum-Zeit-Ausbeute weniger Glykol VI eingesetzt wurde, als für die Cyclisierungsreaktion wünschenswert ist. Vorzugsweise liegt das Glykol in der 2- bis 20-fachen molaren Menge vor, bezogen auf die Dicarbonsaurebausteine der Ester II bis V.

Als Katalysatoren, die sowohl für die Herstellung der glykolischen Lösungen der Ester II bis V als auch für die erfindungsgemäße Cyclisierungsreaktion brauchbar sind, können die üblichen sauren oder basischen Veresterungskatalysatoren dienen, die bekanntlich zugleich Umesterungskatalysatoren sind. Geeignete Katalysatoren sind z.B. unter den Verfahrensbedingungen hinreichend stabile Säuren, wie Schwefelsäure, Natriumhydrogensulfat, Phosphorsäure und Sulfonsäuren; weiterhin Alkalimetalle und Alkalialkoholate; Magnesium-, Mangan-, Cadmium-, Eisen-, Cobalt-, Zinn-, Blei-, Aluminium- und Titanverbindungen. Bevorzugt werden homogen gelöste Katalysatoren vom Typ einer Lewis-Säure. Auch die in der gleichzeitig anhängigen deutschen Patentanmeldung (O.Z. 5276) beschriebenen Eisen(III)-komplexe lassen sich mit Vorteil als Katalysatoren für die vorliegende Erfindung verwenden.

Die für die Herstellung der glykolischen Lösungen der Edukte II bis V verwendeten Katalysatoren können im allgemeinen gleich in der Lösung verbleiben, da sie, wie gesagt, zugleich brauchbare Katalysatoren für die erfindungsgemäße Cyclisierungsreaktion sind. Dies gilt besonders dann, wenn die Katalysatoren homogen gelöst sind. Allerdings kann zusätzlich oder statt dessen Katalysator mit dem inerten hochsiedenden Reaktionsmedium eingebracht werden. Wenn man das Verfahren nach der Erfindung kontinuierlich durchführt, kann man dem zurückgeführten inerten hochsiedenden Medium, das bereits Katalysator enthält, weiteren Katalysator zufügen.

Es hat sich als zweckmäßig erwiesen, wenn in der Verdampfungszone pro Mol monomerer Dicarbonsäure-bis(glykol)ester II bzw. Dicarbonsäurebaustein in den Estern III bis V 0,01 bis 10 Gew.-% Katalysator vorliegen. Wenn man das Verfahren kontinuierlich durchführt, kann man die erwünschte Katalysatorkonzentration in der Verdampfungszone durch die Umlaufgeschwindigkeit des inerten hochsiedenden Mediums regeln. Bei hoher Umlaufgeschwindigkeit ist es möglich, hohe Konzentrationen an Katalysator in der Verdampfungszone bereitzustellen. Dies ist ein Vorteil des erfindungsgemäßen Verfahrens in seiner kontinuierlichen Ausführungsform.

Geeignete inerte hochsiedende. d.h. unter Atmosphärendruck >400°C siedende Medien sind z.B. Glykoldialkylether und Polyalkylenglykoldialkylether. Im einzelnen seien beispielsweise genannt: Polyethylenglykol(1000)-dimethylether (PEG DME 1000), PEG DME 2000, PEG DME 5000, Polyethylenglykol(1000)-diethylether (PEG DEE 1000), PEG DEE 2000 und PEG DEE 5000. Die Zahlen bedeuten die Molekulargewichte,

Neben der Anwesenheit bestimmter molarer Mengen Glykol, bezogen auf den Ester II und die Dicarbonsäurebausteine in den Estern III bis V, ist auch die Gewichtsmenge an inertem hochsiedendem Medium, bezogen auf die Summe der Gewichtsmengen der Ester II bis V, ein wesentliches Merkmal der Erfindung. Als Ergebnis dieser Maßnahmen wird in nur einem Durchgang ein weitgehender Umsatz der Ester II bis V zu dem makrocyclischen Ester I erreicht, so daß bei kontinuierlicher Durchführung des Verfahrens kaum eine Anreicherung an nichtflüchtigen hochpolymeren Estern III bis V (mit x = >10) in dem im Kreis geführten Reaktionsmedium erfolgt. Die benötigte Menge des inerten, hochsiedenden Mediums richtet sich dabei nicht nur nach der in einer Zeiteinheit zugeführten Menge an Estern II bis V sowie Glykol VI, sondern auch nach der optimalen Verweilzeit in der Verdampfungszone. die u.a. von den apparativen Verhältnissen, insbesondere der Art des Verdampfers und der Umwälzleistung der Pumpe, sowie von der Temperatur in der Verdampfungszone abhängt. Auf jeden Fall müssen die relevanten Parameter so aufeinander abgestimmt werden, daß in der Verdampfungszone die 0,1 bis 20-fache, vorteilhaft die 2- bis ca. 10-fache Gewichtsmenge, bezogen auf die Summe der Gewichtsmengen der Ester II bis V, an inertem hochsiedendem Medium vorliegt. Ein größerer Verdünnungsgrad, d.h. eine mehr als 20-fache Menge an inertem hochsiedendem Medium, bringt keinen ökonomischen Vorteil, ist allerdings für den Reaktionsverlauf auch nicht nachteilig.

Als Verdampfer und zugleich Reaktoren eignen sich alle üblichen Verdampfer mit großer Oberfläche, wie Dünnschicht-, Fallfilm-, Rieselfilm- und Kurzwegverdampfer. Die erforderliche Wärmemenge kann in diesen Fällen vorteilhaft direkt über den Verdampfer eingebracht werden. Wird ein Kreislaufstrom des inerten hochsiedenden Mediums über einen Wärmetauscher geführt, so kann die Reaktion auch in einem Rieselbettreaktor stattfinden. Eine andere geeignete Variante ist ein Rührreaktor mit einem Festbettkatalysator, der mit einem üblichen Verdampfer mit großer Oberfläche über eine Kreislaufführung verbunden ist. Schließlich kann man auch das vorerhitzte, Edukt II bis V und Glykol VI sowie Katalysator enthaltende hochsiedende Reaktionsmedium in einen Verdampfer einsprühen, in dem ein erhitztes inertes Trägergas strömt und den makrocyclischen Ester I sowie überschüssiges Glykol VI austrägt, während das hochsiedende Reaktionsmedium aus dem Verdampfer flüssig abläuft. In geeigneten Verdampfern liegt das hochsiedende Reaktionsmedium, welches Edukt II bis V, Glykol VI, Katalysator und nach Fortschreiten der Reaktion auch makrocyclischen Ester I enthält, in dünner Schicht von weniger als 2 cm Dicke, vorteilhaft von weniger als 0,5 cm Dicke oder in Tropfenform vor, bietet also eine große, verdampfungsfördernde spezifische Oberfläche. Dementsprechend kurz sind die Verweilzeiten des hochsiedenden Reaktionsmediums, in dem sich die Umwandlung vom Edukt zum Produkt vollzieht. Großoberflächige Verdampfer gestatten daher bei für den jeweiligen makrocyclischen Ester geeigneten Reaktionstemperaturen einen mindestens 80-prozentigen, vorteilhaft mindestens 90-prozentigen Umsatz des Edukts innerhalb einer Verweilzeit von weniger als 5 Minuten, vorteilhaft von weniger als 2 Minuten. Meistens liegen die Verweilzeiten sogar im Sekundenbereich. Das Destillat trennt sich in allen Fällen in zwei Phasen, wobei der makrocyclische Ester I die obere und das Glykol VI die untere Phase darstellt.

Es ist überraschend, daß die erfindungsgemäße Reaktion gerade in einem Verdampfer mit großer Oberfläche so gut gelingt, weil unter diesen Bedingungen das Glykol, dessen Anwesenheit in einem molaren Überschuß ein wichtiges Merkmal des Verfahrens nach der Erfindung ist, als Stoff mit dem niedrigsten Siedepunkt aller im Reaktionsgemisch vorhandenen Komponenten besonders schnell aus dem Reaktionsgemisch entfernt wird.

Das erfindungsgemäße Verfahren zur Herstellung makrocyclischer Ester führt überraschend selbst dann noch zu hohen, nahezu quantitativen Ausbeuten, wenn neben dem monomeren Ester II und höhermolekularen Estern III bis V (x = 2 bis 10) hochpolymere Ester III bis V (x = >10) in erheblichen Mengen vorliegen. Sollten sich aber nach längerer Zeit in dem im Kreis geführten inerten hochsiedenden Medium unerwünscht große Mengen an hochpolymeren Estern III bis V ansammeln, so können auch diese durch kurzzeitige erhöhte Glykolzufuhr depolymerisiert und in makrocyclischen Ester I überführt werden. Alternativ kann man auch eine Teilmenge des mit hochpolymeren Estern angereicherten inerten hochsiedenden Mediums bei der beschriebenen Herstellung der glykolischen Lösung der Ester II bis V verwenden oder mitverwenden. Auch dabei findet ein Abbau zu höhermolekularen (x = 2 bis 10) Estern III bis V und monomerem Ester II statt.

Die folgenden Beispiele werden gegeben, um das Verfahren nach der Erfindung weiter zu erläutern, und sollen deren Anwendungsbereich nicht einschränken.

### Beispiel 1

X kg oligomerer Dicarbonsäure-bisglykolester der Formel II mit m = 11 und n = 2, die 0,1 Gew.-% des Mono-Na-Salzes des Eisen(III)-Ethylendiamintetraessigsäure-Komplexes enthalten, werden zusammen mit Y kg Ethylenglykol mit Hilfe von Dosierpumpen zur cyclisierenden Depolymerisation einem Reaktor zugeführt, in dem sich Z kg Polyethylenglykol(2000)-dimethylether als hochsiedendes Medium befinden. Der Reaktor besteht aus einem beheizten Fallfilmverdampfer mit ca. 1,5 m² Oberfläche und einer in den Heizkreislauf eingebundenen Sumpfvorlage mit einem Volumen von ca. 50 l sowie einer Rückführung zum Kopf des Fallfilmverdampfers, der eduktseitig mit einem Vorwärmer ausgerüstet ist.

Die Brüden werden über ein Brüdenrohr und einen Kondensator geführt. Der Destillationsvorlage ist ein Trenngefäß nachgeschaltet. Das aus diesem Trenngefäß als obere Phase abgezogene Material ist Ethylenbrassylat mit etwa 5 Gew.-% Ethylenglykol. Durch Destillation ohne nennenswerte Trennleistung wird reines Ethylenbrassylat erhalten. Die untere abgeschiedene Phase ist Ethylenglykol mit 2 bis 3 Gew.-% Ethylenbrassylat, das zusammen mit frischem Ethylenglykol für einen neuen Ansatz verwendet werden kann.

Die Einsatzmengen. Verfahrensbedingungen und Ergebnisse einiger Ansätze gehen aus der folgenden Tabelle 1 hervor.

**Tabelle 1**

| Ansatz | hochs. Medium Z (kg) | p min (hPa) | T max (°C) | | mittlere Verweilzeit am Reaktor (sec) | Menge Y | Menge X | Ausbeute Ethylenbrassylat rein | |
|---|---|---|---|---|---|---|---|---|---|
| | | | | | | | | (kg) | (%) |
| 1.1 | 15 | 20 | 270 | | 180 | 30 | 10 | 9,5 | 95 |
| 1.2 | 15 | 20 | 270 | | 180 | 27 | 13 | 12,1 | 93 |
| 1.3 | 15 | 30 | 280 | | 360 | 38 | 12 | 11,5 | 96 |
| 1.4 | 35 | 30 | 280 | | 360 | 42 | 11 | 10,6 | 96 |
| 1.5 | 35 | 5 | 260 | | 30 | 44 | 14 | 12,9 | 92 |
| 1.6 | 35 | 5 | 260 | | 30 | 64 | 16 | 14,9 | 93 |

### Beispiel 2

X kg oligomerer Dicarbonsäurebisglykolester der Formel II mit m = 11 und n = 2, die 0,1 Gew.-% eines Eisen(III)-komplex-Katalysators (Eisen(III)-acetylacetonat) enthalten, werden zusammen mit Y kg Ethylenglykol mit Hilfe von Dosierpumpen zur cyclisierenden Depolymerisation einem Reaktor zugeführt, in dem sich Z kg Polyethylenglykol(2000)-dimethylether als hochsiedendes Medium befinden. Zur Verbesserung der Reaktionsgeschwindigkeit werden dem hochsieden Medium ca 0,1 Gew.-% des Katalysators zugesetzt. Der Reaktor besteht aus einem 250 l Rührkessel, auf den ein beheizter Fallfilmverdampfer mit ca. 1,0 m² Oberfläche aufgesetzt ist. Das Sumpfprodukt des Reaktors wird mit Hilfe einer Wälzpumpe auf den Fallfilmverdampfer zurückgeführt, der die Verdampfungsleistung gegenüber einer einfachen Destillation aus dem Kessel erhöht.

Die Brüden werden über ein Brüdenrohr und einen Kondensator geführt. Der Destillationsvorlage ist ein Trenngefäß nachgeschaltet. Das aus diesem Trenngefäß als obere Phase abgezogene Material ist Ethylenbrassylat mit etwa 5 Gew.-% Ethylenglykol. Durch Destillation ohne nennenswerte Trennleistung wird reines Ethylenbrassylat erhalten. Die untere abgeschiedene Phase ist Ethylenglykol mit 2 bis 3 Gew.-% Ethylenbrassylat, das zusammen mit frischem Ethylenglykol für einen neuen Ansatz verwendet werden kann.

Die Einsatzmengen. Verfahrensbedingungen und Ergebnisse einiger Ansätze gehen aus der folgenden Tabelle 2 hervor.

**Tabelle 2**

| Ansatz | hochs. Medium Z (kg) | p min (hPa) | T max (°C) | mittlere Verweilzeit am Reaktor (sec) | Menge Y | Menge X | Ausbeute Ethylenbrassylat rein | |
|---|---|---|---|---|---|---|---|---|
| | | | | | | | (kg) | (%) |
| 2.1 | 105 | 20 | 270 | 160 | 35 | 11 | 10,0 | 91 |
| 2.2 | 125 | 20 | 270 | 540 | 29 | 10 | 9,2 | 92 |
| 2.3 | 125 | 30 | 280 | 1.080 | 48 | 12 | 11,5 | 96 |
| 2.4 | 145 | 30 | 280 | 1.080 | 50 | 11,5 | 11,2 | 97 |
| 2.5 | 145 | 5 | 270 | 90 | 46 | 11 | 10,5 | 99 |
| 2.6 | 155 | 5 | 260 | 90 | 67 | 16,5 | 5,5 | 94 |

### Beispiel 3

X kg oligomerer Dicarbonsäurebisglykolester der Formel II mit m = 10 und n = 2, die 0,1 Gew.-% eines Eisen(III)-komplex-Katalysators (K₃[Fe(CN)₆]) enthalten, werden zusammen mit Y kg Ethylenglykol mit Hilfe von Dosierpumpen zur cyclisierenden Depolymerisation einem Reaktor zugeführt, in dem sich Z kg Polyethylenglykol(2000)-dimethylether als hochsiedendes Medium befinden. Der Reaktor besteht aus einem beheizten Fallfilmverdampfer mit ca. 1,5 m² Oberfläche und einer in den Heizkreislauf eingebundenen Sumpfvorlage mit einem Volumen von ca. 50 l sowie einer Rückführung zum Kopf des Fallfilmverdampfers, der eduktseitig mit einem Vorwärmer ausgerüstet ist.

Die Brüden werden über ein Brüdenrohr und einen Kondensator geführt. Der Destillationsvorlage ist ein Trenngefäß nachgeschaltet. Das aus diesem Trenngefäß als obere Phase abgezogene Material ist Ethylenglykoldodecandioat mit etwa 4 Gew.-% Ethylenglykol. Durch Destillation ohne nennenswerte Trennleistung wird reines Ethylenglykoldodecanodiat erhalten. Die untere abgeschiedene Phase ist Ethylenglykol mit 2 bis 3 Gew.-% Ethylenglykoldodecanodiat, das zusammen mit frischem Ethylenglykokol für einen neuen Ansatz verwendet werden kann.

Die Einsatzmengen, Verfahrensbedingungen und Ergebnisse einiger Ansätze gehen aus der folgenden Tabelle 3 hervor.

**Tabelle 3**

| Ansatz | hochs. Medium Z (kg) | p min (hPa) | T max (°C) | mittlere Verweilzeit am Reaktor (sec) | Menge Y | Menge X | Ausbeute Ethylenbrassylat rein | |
|---|---|---|---|---|---|---|---|---|
| | | | | | | | (kg) | (%) |
| 3.1 | 25 | 20 | 270 | 190 | 35 | 11 | 12,0 | 94 |
| 3.2 | 25 | 20 | 275 | 120 | 27 | 6,5 | 6,0 | 92 |
| 3.3 | 15 | 30 | 280 | 330 | 18 | 4,5 | 4,2 | 93 |
| 3.4 | 35 | 30 | 280 | 300 | 42 | 10,5 | 10,2 | 97 |
| 3.5 | 35 | 15 | 260 | 40 | 64 | 12 | 10,5 | 89 |
| 3.6 | 35 | 15 | 270 | 50 | 70 | 19 | 17,0 | 90 |

### Beispiel 4

X kg oligomerer Dicarbonsäurebisglykolester der Formel II mit m = 10 und n = 2, die 0,1 Gew.-% des Monokaliumsalzes des Eisen(III)-Ethylendiamintetraessigsäure-Komplexes enthalten, werden zusammen mit Y kg Ethylenglykol mit Hilfe von Dosierpumpen zur cyclisierenden Depolymerisation einem Reaktor zugeführt, in dem sich Z kg Polyethylenglykol(2000)-dimethylether als hochsiedendes Medium befinden. Der Reaktor besteht aus einem beheizten Fallfilmverdampfer mit ca. 1,5 m² Oberfläche und einer in den Heizkreislauf eingebundenen Sumpfvorlage mit einem Volumen von ca. 50 l. Im Gegensatz zu den Beispielen 1 und 3 wurde der Sumpfablauf nicht zum Kopf des Fallfilmverdampfers zurückgeführt.

Die Brüden werden über ein Brüdenrohr und einen Kondensator geführt. Der Destillationsvorlage ist ein Trenngefäß nachgeschaltet. Das aus diesem Trenngefäß als obere Phase abgezogene Material ist Ethylenglykoldodecanodiat mit etwa 4 Gew.-% Ethylenglykol. Durch Destillation ohne nennenswerte Trennleistung wird reines Ethylenglykoldodecandioat erhalten. Die untere abgeschiedene Phase ist Ethylenglykol mit 2 bis 3 Gew.-% Ethylenglykoldodecanodiat, das zusammen mit frischem Ethylenglykokol für einen neuen Ansatz verwendet werden kann.

Die Einsatzmengen, Verfahrensbedingungen und Ergebnisse einiger Ansätze gehen aus der folgenden Tabelle 3 hervor.

**Tabelle 4**

| Ansatz | hochs. Medium Z (kg) | p min (hPa) | T max (°C) | mittlere Verweilzeit am Reaktor (sec) | Menge Y | Menge X | Ausbeute Ethylenbrassylat rein | |
|---|---|---|---|---|---|---|---|---|
| | | | | | | | (kg) | (%) |
| 4.1 | 25 | 20 | 270 | 190 | 35 | 11 | 5,0 | 46 |
| 4.2 | 25 | 20 | 275 | 120 | 27 | 6,5 | 2,5 | 39 |
| 4.3 | 15 | 30 | 280 | 330 | 18 | 4,5 | 2,9 | 64 |
| 4.4 | 35 | 30 | 280 | 300 | 42 | 10,5 | 6,4 | 61 |
| 4.5 | 35 | 15 | 260 | 40 | 64 | 12 | 3,7 | 31 |
| 4.6 | 35 | 15 | 270 | 50 | 70 | 19 | 5,3 | 28 |

Die Ausbeute bezieht sich hier wie in den vorangehenden Beispielen auf eingesetzten höhermolekularen Ester, vernachlässigt also dessen im Sumpfablauf verbliebenen nicht umgesetzten Anteil. Das Beispiel zeigt die vorteilhafte Wirkung der Kreislaufführung, die in den Beispielen 1 bis 3 angewandt wurde und zu einer Umsetzung auch dieser Anteile führt.

## Patentansprüche

1. Verfahren zur Herstellung von makrocyclischen Estern der allgemeinen Formel 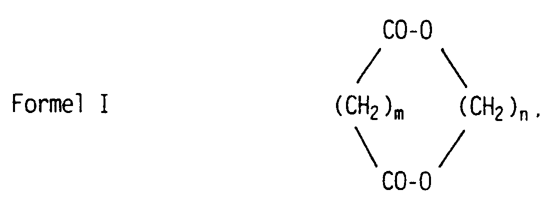 in der m die Bedeutung einer ganzen Zahl von 6 bis 14 hat und n für eine ganze Zahl von 2 bis 6 steht, dadurch gekennzeichnet, daß man
(a) Dicarbonsäure-bis(glykol)ester der allgemeinen Formeln
HO-(CH₂)ₙ-O-CO-(CH₂)ₘ-CO-O-(CH₂)ₙ-OH, Formel II
H[O-(CH₂)ₙ-O-CO-(CH₂)ₘ-CO-]ₓ-O-(CH₂)ₙ-OH, Formel III
H[O-(CH₂)ₙ-O-CO-(CH₂)ₘ-CO]ₓ-OH Formel IV
und/oder
HO-CO-(CH₂)ₘ-CO-[O-(CH₂)ₙO-CO-(CH₂)ₘ-CO-]ₓ-OH, Formel V
in denen m und n die für die Formel I angegebenen Bedeutungen haben und x für eine ganze Zahl >1 steht,
(b) ein Glykol der allgemeinen Formel
HO-(CH₂)ₙ-OH, Formel VI
in der n denselben numerischen Wert wie in den Formeln I bis V hat, in der 1- bis 50-fachen molaren Menge, bezogen auf den Ester II und die Dicarbonsäurebausteine der Ester III bis V, und
(c) ein inertes hochsiedendes Reaktionsmedium in der 0,1 bis 20-fachen Gewichtsmenge, bezogen auf die Summe der Gewichtsmengen der Ester II bis V, in Gegenwart
(d) eines Katalysators
in einem Verdampfer mit großer Oberfläche auf Temperaturen von 150 bis 350°C bei einem vermindertem Druck von etwa 0,1 bis etwa 500 mbar erhitzt, wodurch unter Abspaltung von Glykol der makrocyclische Ester I entsteht, der zusammen mit Glykol VI abdestilliert und durch Kondensation gewonnen wird.

2. Verfahren nach Anspruch 1, dadurch gekennzeichnet, daß das Verfahren kontinuierlich durchgeführt wird, wobei das inerte hochsiedende Medium, das den Katalysator und nicht umgesetzte Ester II bis V enthält, im Kreis geführt und dem Kreislauf frischer Ester II bis V sowie frisches Glykol VI zugesetzt wird.

3. Verfahren nach Anspruch 1 oder 2, dadurch gekennzeichnet, daß das Glykol in der 2- bis 20-fachen molaren Menge, bezogen auf die Dicarbonsäurebausteine in den Estern II bis V, eingesetzt wird.

4. Verfahren nach einem der Ansprüche 1 bis 3, dadurch gekennzeichnet, daß die Temperatur 180 bis 300°C beträgt.

5. Verfahren nach einem der Ansprüche 1 bis 3, dadurch gekennzeichnet, daß die Temperatur 200 bis 280°C beträgt.

6. Verfahren nach einem der Ansprüche 1 bis 5, dadurch gekennzeichnet, daß der Druck 0,5 bis 100 mbar beträgt.

7. Verfahren nach einem der Ansprüche 1 bis 6, dadurch gekennzeichnet, daß als inertes hochsiedendes Medium (c) ein oder mehrere Lösemittel aus der Gruppe der inerten höheren Glykoldialkylether und Polyalkylenglykoldialkylether verwendet wird bzw. werden.

8. Verfahren nach einem der Ansprüche 1 bis 7, dadurch gekennzeichnet, daß das inerte hochsiedende Medium (c) in der Reaktionszone in der 1- bis 15-fachen Gewichtsmenge, bezogen auf die Summe der Gewichtsmengen der Ester II bis V, vorliegt.

9. Verfahren nach einem der Ansprüche 1 bis 7, dadurch gekennzeichnet, daß das inerte hochsiedende Medium (c) in der Reaktionszone in der 2- bis 10-fachen Gewichtsmenge, bezogen auf die Summe der Gewichtsmengen der Ester II bis V, vorliegt.

10. Verfahren nach einem der Ansprüche 1 bis 9, dadurch gekennzeichnet, daß die Ester II bis V sowie das Glykol VI in Form einer glykolischen Lösung der Ester eingesetzt werden, die durch Veresterung der Dicarbonsäure mit überschüssigem Glykol, durch Umesterung eines Dicarbonsäuredialkylesters mit Alkylresten mit 1 bis 6 Kohlenstoffatomen mit überschüssigem Glykol oder durch Depolymerisation von hochpolymeren Estern III bis V (x = >10) mit überschüssigem Glykol entstanden ist.

11. Verfahren nach einem der Ansprüche 1 bis 10, dadurch gekennzeichnet, daß man als Katalysator (d) einen der üblichen sauren oder basischen Veresterungs- oder Umesterungskatalysatoren verwendet.

12. Verfahren Anspruch 11, dadurch gekennzeichnet, daß man als Katalysator (d) Schwefelsäure, Phosphorsäure oder eine Sulfonsäure verwendet.

13. Verfahren Anspruch 11, dadurch gekennzeichnet, daß man als Katalysator (d) ein Alkalimetall oder -alkoholat verwendet.

14. Verfahren Anspruch 11, dadurch gekennzeichnet, daß man als Katalysator (d) eine oder mehrere Magnesium-, Mangan-, Cadmium-, Eisen-, Cobalt-, Zinn-, Blei-, Aluminium- oder Titanverbindungen verwendet.

15. Verfahren nach Anspruch 10, dadurch gekennzeichnet, daß für die Veresterung bzw. Umesterung derselbe Katalysator verwendet wird, der später als Katalysator (d) dient.

16. Verfahren nach einem der Ansprüche 2 bis 15, dadurch gekennzeichnet, daß man dem im Kreis geführten inerten hochsiedendem Medium Katalysator (d) zusetzt.

17. Verfahren nach einem der Ansprüche 1 bis 15, dadurch gekennzeichnet, daß man als Verdampfer mit großer Oberfläche einen wie Dünnschicht-, Fallfilm- und Kurzwegverdampfer verwendet.

18. Verfahren nach einem der Ansprüche 1 bis 15, dadurch gekennzeichnet, daß man als Verdampfer mit großer Oberfläche das Rieselbett einer Kolonne verwendet.
